# EUROPEAN PATENT APPLICATION

(11) **EP 3 884 927 A1**
(43) Date of publication of application: **29.09.2021**
(21) Application number: 19887729.2
(22) Date of filing: 19.11.2019
(51) Int. Cl.: A61K 8/19, A61K 8/29, A61K 8/27, A61Q 17/04

(54) **ULTRAVIOLET SHIELDING AGENT COMPOSITION COMPRISING CERIUM OXIDE PARTICLES HAVING SURFACE MODIFIED WITH POLYHYDROXYSTEARIC ACID**

(30) Priority: 19.11.2018 KR 20180142928
(71) Applicant: Soulbrain Co., Ltd., Seongnam-si, Gyeonggi-do 13486 (KR)
(72) Inventor: LEE, Jeong Ho, Seongnam-si Gyeonggi-do 13486 (KR); KIM, Seok Joo, Seongnam-si Gyeonggi-do 13486 (KR)
(74) Representative: M. Zardi & Co S.A.
(86) International application number: PCT/KR2019/015888
(87) International publication number: WO 2020/106039

(57) **Abstract**

Disclosed are a sunscreen composition containing cerium oxide (CeO₂) particles having surfaces modified with polyhydroxystearic acid and a method for preparing the sunscreen composition. The sunscreen composition containing cerium oxide (CeO₂) particles having surfaces modified with polyhydroxystearic acid exhibits excellent dispersion stability even after a long period of time elapses.

## Description

### [Technical Field]

The present invention relates to a sunscreen composition containing cerium oxide (CeO₂) particles having surfaces modified with polyhydroxystearic acid and a method for preparing the sunscreen composition.

### [Background Art]

Since the first cosmetic product containing a sunscreen agent was developed in the United States in 1928, the demand for sunscreen agents has steadily increased. Sunscreen agents are intended to prevent skin cancer, sunburn, and photoaging caused by ultraviolet rays. Recently, interest in prevention of photoaging through blocking of ultraviolet rays corresponding to UVA1 and UVA2 wavelengths is increasing for cosmetic purposes. Ultraviolet blocking functions are imparted to most formulations such as BB cream, CC cream, cushion, sun spray, and sun stick in addition to sun cream.

In order to block ultraviolet rays, sunscreen agents are added, and sunscreen agents may be divided into organic sunscreen agents and inorganic sunscreen agents. As organic sunscreen agents, there are typically chemical sunscreen agents that convert light into heat. As inorganic sunscreen agents, there are typically physical sunscreen agents that reflect, scatter, and absorb light. Unlike basic skin care cosmetics, sunscreen is mainly used to attenuate ultraviolet rays on the upper part of the epidermis, that is, the outermost part of the skin. However, in the case of organic sunscreen agents such as avobenzone, the molecular size thereof is small and there is a possibility that the organic sunscreen agent penetrates the skin. Organic sunscreen agents have advantages of having little white turbidness and various absorption wavelengths but may cause skin problems or side effects such as irritation of the eyes when applied around the eyes in the case of sensitive skin. On the other hand, inorganic sunscreen agents are relatively safe and have a favorable blocking effect but are white pigments having high refractive indices, and thus may cause problems such as white turbidness. Due to the recent nature-friendly trend of cosmetic materials, in Korea, the preference for sunscreen products of 'inorganic sunscreen' formulations containing only inorganic sunscreen agents as functional ingredients is high.

Titanium dioxide (TiO₂) and zinc oxide (ZnO) are used as inorganic sunscreen agents but have various disadvantages. First, the energy band gaps of titanium dioxide and zinc oxide are 3.0 eV and 3.2 eV, respectively, and thus titanium dioxide and zinc oxide are advantageous for UVB and UVA2 absorption but cannot absorb UVA1 that is an intermediate wavelength. Second, the refractive indices of titanium dioxide and zinc oxide are as high as 2.7 and 2.2, respectively, and thus white cloudy appearance may be noticeable when the sunscreen is applied to the skin. Third, titanium dioxide and zinc oxide have a great photocatalytic effect to decompose or denature organic materials, particularly coloring matters, under light energy and thus may cause ingredient denaturation of the formulation and pigmentation. In particular, when the photocatalytic effect is great, the surfaces of titanium dioxide and zinc oxide are required to be covered with a second material for safety reasons. In the case of titanium dioxide, aluminum oxide (Al₂O₃) or silicon dioxide (SiO₂) is used to cover 20 parts by weight or more of titanium dioxide. However, when the surface of titanium dioxide is covered with aluminum oxide and silicon dioxide, there may be disadvantages that the powder texture is heavy, the sunscreen is not smoothly applied, and the feel of use is stiff. Hence, it is required to develop a sunscreen composition that can compensate for the above disadvantages.

Accordingly, the present inventors have studied to solve the above problems, found out that a sunscreen composition which can absorb UVA1, suppresses white turbidness by a low refractive index, and is stable because of a low photocatalytic effect can be formed when cerium oxide having the surface modified with a fatty acid is used in the sunscreen composition, and applied for this sunscreen composition (Korean Patent Application No. 10-2017-0142617).

However, in the case of a sunscreen composition containing cerium oxide having surfaces modified with a fatty acid as described above, a problem has arisen that the dispersion stability is deteriorated over time. As a result of further studies, it has been confirmed that the sunscreen composition exhibits excellent dispersion stability even after a long period of time elapses when the surface of cerium oxide is modified with polyhydroxystearic acid instead of a fatty acid, and the present invention has been thus completed.

In this regard, Korean Patent Registration No. 10-1600058 discloses a metal oxide conjugated highly flat cellulose powder and a cosmetic containing the same.

### [Summary of Invention]

### [Technical Problem]

The present invention has been devised to solve the above-described problems, and an embodiment of the present invention provides a sunscreen composition containing cerium oxide (CeO₂) particles having surfaces modified with polyhydroxystearic acid.

Another embodiment of the present invention provides a method for preparing cerium oxide (CeO₂) particles having surfaces modified with polyhydroxystearic acid.

Still another embodiment of the present invention provides a method for preparing the sunscreen composition.

The technical problem to be achieved by the present invention is not limited to the technical problems mentioned above, and other technical problems that are not mentioned will be clearly understood by those skilled in the technical field to which the present invention pertains from the following description.

### [Solution to Problem]

As a technical means for achieving the above-described technical problems, an aspect of the present invention provides a sunscreen composition containing cerium oxide (CeO₂) particles having surfaces modified with substituted or unsubstituted polyhydroxystearic acid.

The polyhydroxystearic acid may have a hydroxystearic acid repeating unit.

The polyhydroxystearic acid may further have a crosslinking repeating unit.

The polyhydroxystearic acid may have a weight average molecular weight of 1,000 g/mol to 100,000 g/mol.

The content of the polyhydroxystearic acid may be 1 part by weight to 10 parts by weight with respect to 100 parts by weight of cerium oxide particles.

The content of the surface-modified cerium oxide particles may be 1 part by weight to 50 parts by weight with respect to 100 parts by weight of the sunscreen composition.

The primary particle size of the cerium oxide may be 10 to 30 nm, the secondary particle size of the cerium oxide may be 100 to 500 nm, and the ratio of the secondary particle size to the primary particle size may be 5 to 50.

The sunscreen composition may further contain one or more kinds of particles selected from the group consisting of titanium oxide particles and zinc oxide particles.

The content of the titanium oxide particles or zinc oxide particles may be 1 part by weight to 25 parts by weight with respect to 100 parts by weight of the sunscreen composition.

Another aspect of the present invention provides a method for preparing cerium oxide particles having surfaces modified with polyhydroxystearic acid, the method including: mixing polyhydroxystearic acid and a solvent to prepare a mixed solution; and mixing the mixed solution with cerium oxide particles and performing milling to prepare cerium oxide particles having surfaces modified with polyhydroxystearic acid.

The solvent may be a solvent selected from the group consisting of alkyl benzoate, methylene chloride, xylene, dimethylformamide (DMF), nitrobenzene, methylethylketone, dimethicone, polydimethylsiloxane, cyclopentasiloxane, and combinations thereof.

The mixing ratio of the polyhydroxystearic acid to the solvent may be 1:10 to 100.

The mixing ratio of the mixed solution to the cerium oxide particles may be 1:0.5 to 1.5.

Still another aspect of the present invention provides a method for preparing a sunscreen composition, the method including: dispersing the cerium oxide particles prepared to have surfaces modified with polyhydroxystearic acid, in an organic solvent; and mixing the organic solvent with a material selected from the group consisting of silicone oil, a fiber, an emulsifier, a moisturizing agent, a plasticizer, purified water, and combinations thereof.

### [Advantageous Effects of Invention]

According to an embodiment of the present invention, the sunscreen composition containing cerium oxide (CeO₂) particles having surfaces modified with polyhydroxystearic acid may exhibit excellent dispersion stability since the layer separation thereof does not occur even after a long period of time elapses.

In addition, the sunscreen composition has a high dynamic viscosity in the low frequency region and the high frequency region and thus the formulation thereof exhibits excellent emulsification/dispersion phase-stability and the sunscreen composition exhibits excellent application property and thus can be usefully used as a cosmetic composition for ultraviolet blocking.

The sunscreen composition according to an embodiment of the present invention is capable of absorbing UVA1 of the intermediate ultraviolet wavelength region and thus can absorb ultraviolet rays in the entire UVA1 to UVA2 region, so the sunscreen composition has a high sun protection factor and thus has an excellent ultraviolet blocking effect.

The sunscreen composition according to an embodiment of the present invention does not cause white cloudy appearance when applied to the skin since the particles have a low light refractive index, and thus can be used as a cosmetic composition for ultraviolet blocking that provides natural impression of color.

As the surfaces of the cerium oxide particles are modified with polyhydroxystearic acid, the sunscreen composition according to an embodiment of the present invention exhibits high emulsifying property in oil-in-water, water-in-oil, and non-aqueous formulations and can be thus used to prepare various formulations of cosmetic compositions for ultraviolet blocking.

The effects of the present invention are not limited to the above effects, and should be understood to include all effects that can be deduced from the configuration of the invention described in the detailed description or claims of the present invention.

### [Brief Description of Drawings]

FIG. 1 is a schematic diagram illustrating a method for preparing cerium oxide particles having surfaces modified with polyhydroxystearic acid according to an embodiment of the present invention;
FIG. 2 is a schematic diagram illustrating a method for preparing a sunscreen composition according to an embodiment of the present invention;
FIG. 3A is a photograph of a sunscreen composition containing cerium oxide particles having surfaces modified with a fatty acid according to Comparative Example of the present invention;
FIG. 3B is a photograph of a sunscreen composition containing cerium oxide particles having surfaces modified with polyhydroxystearic acid according to Example of the present invention;
FIG. 4 is a graph illustrating the particle size distribution of cerium oxide particles having surfaces modified with polyhydroxystearic acid according to Example of the present invention; and
FIG. 5 is a graph illustrating the thermogravimetric analysis results of cerium oxide particles depending on the amount of polyhydroxystearic acid added according to
Example of the present invention.

### [Description of Embodiments]

Hereinafter, the present invention will be described in more detail. However, the present invention can be implemented in several different forms. The present invention is not limited by the embodiments described herein, and the present invention is only defined by the claims to be described later.

In addition, terms used in the present invention are used only to describe specific embodiments, and are not intended to limit the present invention. Singular expressions include plural expressions unless the context clearly indicates otherwise. In the entire specification of the present invention, "including" a certain component means that other components may be further included, rather than excluding other components unless specifically stated to the contrary.

A first aspect of the present application provides a sunscreen composition containing cerium oxide (CeO₂) particles having surfaces modified with substituted or unsubstituted polyhydroxystearic acid.

Hereinafter, the sunscreen composition according to the first aspect of the present application will be described in detail.

In an embodiment of the present application, the polyhydroxystearic acid may have a hydroxystearic acid repeating unit. At this time, the hydroxystearic acid may have a structure represented by the following Chemical Formula (1).

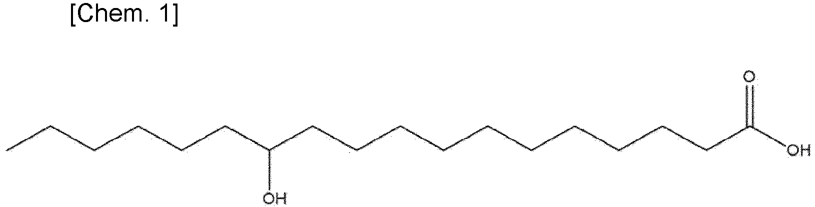

In an embodiment of the present application, the hydroxystearic acid repeating unit may have a structure represented by the following Chemical Formula (2).

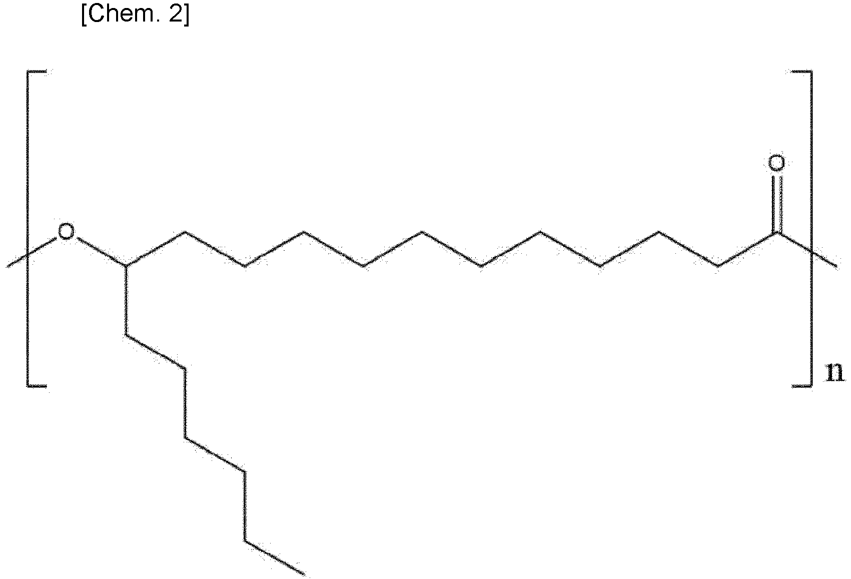

In other words, the polyhydroxystearic acid may be formed by dehydration polymerization reaction of a hydroxyl group and a carboxyl group between hydroxystearic acid monomers in a solvent such as xylene. At this time, the dehydration polymerization reaction may be performed at a high temperature of about 200°C.

In an embodiment of the present application, the polyhydroxystearic acid may have a weight average molecular weight of 1,000 g/mol to 100,000 g/mol. When the weight average molecular weight of polyhydroxystearic acid is less than 1,000 g/mol, the particle surface may not be sufficiently treated and the dispersion stability of the sunscreen composition may be deteriorated. When the weight average molecular weight exceeds 100,000 g/mol, the molecular weight of polyhydroxystearic acid with respect to that of the cerium oxide particles increases and thus the ultraviolet blocking effect by the sunscreen composition may decrease.

In an embodiment of the present application, the cerium oxide particles may be prepared from a cerium precursor such as cerium hydroxide, cerium carbonate, cerium nitrate, cerium chloride, or ammonium cerium nitrate. All cerium oxide particles prepared by ordinary cerium oxide preparation methods may be used without particular limitation. The cerium oxide particles may be in the form of a plate shape, a flake shape, or a spherical shape, and there is no particular limitation on the form of cerium oxide particles.

In an embodiment of the present application, the primary particle size of the cerium oxide (CeO₂) particles may be 3 to 35 nm, 5 to 32 nm, or preferably 10 to 30 nm. The secondary particle size of the cerium oxide (CeO₂) particles may be 50 to 600 nm, 80 to 550 nm, or preferably 100 to 500 nm. The ratio of the secondary particle size to the primary particle size may be 1 to 55, 3 to 53, or preferably 5 to 50.

In an embodiment of the present application, as the cerium oxide particles are contained in the sunscreen composition according to the present invention, the cerium oxide particles can absorb the wavelengths in the UVA1 region that is the intermediate ultraviolet wavelength region and thus can play a role of widening the ultraviolet blocking and absorption region of the sunscreen composition.

In an embodiment of the present application, by modifying the surfaces of the cerium oxide particles with polyhydroxystearic acid, the sunscreen composition may exhibit excellent emulsification/dispersion phase-stability and excellent application property. In other words, the surface is modified with polyhydroxystearic acid that is a hydrophobic polymer, and thus the emulsification smoothly proceeds and the dispersion stability of the formulation can be excellent.

In an embodiment of the present application, the content of polyhydroxystearic acid may be 0.3 part by weight to 15 parts by weight, preferably 0.5 part by weight to 11 parts by weight, more preferably 1 part by weight to 10 parts by weight with respect to 100 parts by weight of cerium oxide particles. When the surface is modified with less than 0.3 part by weight of polyhydroxystearic acid, the whole surfaces of the cerium oxide particles may not be modified, and thus problems of emulsification/dispersion may occur at the time of cosmetic preparation using a solvent. When the surface is modified with more than 15 parts by weight of polyhydroxystearic acid, the excess polyhydroxystearic acid polymers that have not been used to cover the particle surfaces gather together and may act as impurities at the time of cosmetic preparation. When the content of polyhydroxystearic acid is too low, the particle surfaces may not be sufficiently covered. When the content of polyhydroxystearic acid is too high, the polyhydroxystearic acid polymers gather as excess molecules and act as impurities at the time of cosmetic preparation. This may lead to rancidity of the cosmetic, and excessive oiliness may diminish the feel of use.

In an embodiment of the present application, in the sunscreen composition, by modifying the surfaces of the cerium oxide particles with polyhydroxystearic acid, the cerium oxide particles that are hydrophobic particles may exhibit excellent emulsification/dispersion phase-stability.

In an embodiment of the present application, the content of the surface-modified cerium oxide particles may be 1 part by weight to 50 parts by weight, preferably, 2 parts by weight to 40 parts by weight, more preferably 5 parts by weight to 20 parts by weight with respect to 100 parts by weight of the sunscreen composition. When the content of the surface-modified cerium oxide particles is less than 1 part by weight, the content of cerium oxide particles is too low, the wavelengths in the UVA1 region may not be absorbed, and thus the effects of the sunscreen composition according to the present invention may not be exerted. When the content of the surface-modified cerium oxide particles exceeds 50 parts by weight, the solid content is too high, the viscosity of the cosmetic may become too high, and thus the application property may be impaired. When the content of the surface-modified cerium oxide particles is less than 1 part by weight, it may be difficult to expect the ultraviolet blocking effect.

In an embodiment of the present application, the sunscreen composition may further contain one or more kinds of particles selected from the group consisting of titanium oxide particles and zinc oxide particles.

In an embodiment of the present application, the content of the titanium oxide particles or zinc oxide particles may be 1 part by weight to 25 parts by weight with respect to 100 parts by weight of the sunscreen composition. At this time, the titanium oxide particles and zinc oxide particles may be used without particular limitation depending on the purpose and use to be applied as long as the effects of the present invention are not impaired.

In an embodiment of the present application, the sunscreen composition may further contain an organic solvent, silicone oil, a fiber, an emulsifier, a moisturizing agent, a plasticizer, or purified water.

In an embodiment of the present application, as the organic solvent, alcohols, glycols, silicone oil, natural oil, vegetable oil, nut oil, mineral oil and the like may be used. The organic solvent serves as a solvent for dispersing the cerium oxide particles having surfaces modified with polyhydroxystearic acid. The organic solvent may disperse cerium oxide particles having surfaces modified with polyhydroxystearic acid, and all organic solvents suitable for preparation of cosmetic compositions in formulations providing favorable application property may be used without particular limitation.

In an embodiment of the present application, as the silicone oil, dimethicone, cetyl dimethicone, cyclopentasiloxane, cyclohexasiloxane, stearyl dimethicone and the like may be used. The silicone oil may form an oil phase when a cosmetic is emulsified, and plays a role of improving the feel of use.

In an embodiment of the present application, as the fiber, VGL silk and the like may be used. The fiber plays a role of improving the feel of use of the sunscreen composition.

In an embodiment of the present application, as the emulsifier, a PEG silicone emulsifier, a nonionic W/O emulsifier, a cationic emulsifier, an anionic emulsifier and the like may be used. The emulsifier allows each ingredient of the sunscreen composition according to the present invention to be emulsified. The emulsifier plays a role of improving the stability of the formulation by trapping the particles in the emulsion particles in the oil phase.

In an embodiment of the present application, as the moisturizing agent, natural moisturizing factors (NMF) such as polyols including 1,2-hexanediol, glycerin, propylene glycol, butylene glycol, polyethylene glycol, sorbitol, or trehalose, amino acids, urea, lactates, or PCA-Na, high molecular weight moisturizing agents such as hyaluronates, chondroitin sulfate, or hydrolyzed collagen, and the like may be used. The moisturizing agent may increase the moisturizing power of the sunscreen composition according to the present invention and at the same time serve as a preservative.

In an embodiment of the present application, as the plasticizer, DPG (dipropylene glycol) and the like may be used.

In an embodiment of the present application, in addition to the ingredients described above, ingredients usually blended in cosmetic compositions, such as fats and oils, waxes, surfactants, thickeners, coloring matters, cosmetic additives, powders, saccharides, antioxidants, buffers, various extracts, stabilizers, preservatives, and fragrances, may be appropriately blended in the sunscreen composition as long as the effects of the present invention are not impaired.

In an embodiment of the present application, as the fats and oils, vegetable oils such as evening primrose oil, rosehip oil, castor oil, or olive oil, animal oils such as mink oil or squalene, mineral oils such as liquid paraffin or petrolatum, synthetic oil such as silicone oil or isopropyl myristate, and the like may be used.

In an embodiment of the present application, as the waxes, vegetable waxes such as carnauba wax, candelilla wax, or jojoba oil, animal waxes such as beeswax or lanolin, and the like may be used.

In an embodiment of the present application, as the surfactants, an anionic surfactant, a cationic surfactant, an amphoteric surfactant, a nonionic surfactant and the like may be used.

In an embodiment of the present application, as the thickeners, for example, a water-soluble polymer may be used.

In an embodiment of the present application, as the water-soluble polymers, a plant-based (polysaccharide-based) natural polymer such as guar gum, locust bean gum, queen's seed, carrageenan, galactan, gum arabic, tragacanth gum, pectin, mannan, or starch, a microbial (polysaccharide-based) natural polymer such as xanthan gum, dextran, succinolglucan, cadran, or hyaluronic acid, an animal-based (protein-based) natural polymer such as gelatin, casein, albumin, or collagen, a cellulose-based semisynthetic polymer such as methyl cellulose, ethyl cellulose, hydroxy ethyl cellulose, hydroxypropyl cellulose, carboxymethyl cellulose, or methyl hydroxypropyl cellulose, a starch-based semisynthetic polymer such as soluble starch, carboxymethyl starch, or methyl starch, an alginic acid-based semisynthetic polymer such as propylene glycol alginate or alginates, semisynthetic polymers of other polysaccharide derivatives, a vinyl-based synthetic polymer such as polyvinyl alcohol, polyvinylpyrrolidone, polyvinyl methyl ether, carboxyvinyl polymer, or sodium polyacrylate, other synthetic polymers such as polyethylene oxide and ethylene oxide or propylene oxide block copolymer, inorganic substances such as bentonite, laponite, fine silicon oxide, and colloidal alumina, and the like may be used.

In an embodiment of the present application, as the coloring matters, for example, a synthetic coloring matters or natural coloring matters may be used. As the synthetic coloring matters, water-soluble/oil-soluble dyes such as FD&C Yellow No. 6 or FD&C Red No. 4, inorganic pigments such as iron oxide or ultramarine, organic pigments such as D&C Red No. 30 or D&C Red No. 36, lakes such as FD&C Yellow No. 6 Al lake, and the like may be used. As the natural coloring matters, a carotenoid-based coloring matter such as β-carotene, β-apo-8-carotenal, lycopene, capsanthin, bixin, crocin, or canthaxanthin, a flavonoid-based coloring matter such as shisonin, lamanin, niacin, carsamin, safrole yellow, rutin, quercetin, or cacao pigment, a flavin-based coloring matter such as riboflavin, a quinone-based coloring matter such as laccaic acid, carminic acid (cochineal), kermesic acid, alizarin, cichoriin, arkanine, or nikinochrome, a porphyrin-based coloring matter such as chlorophyll or hemoglobin, a diketone-based coloring matter such as circumin (turmeric), a beta-cyanidin-based coloring matter such as betanin, and the like may be used.

In an embodiment of the present application, as the cosmetic additives, for example, vitamins, plant extracts, or animal extracts may be used. Retinol (vitamin A), tocopherol (vitamin E), ascorbic acid (vitamin C) and the like may be used as the vitamins. As the plant extracts, menthol (mint), azulene (chamomile), allantoin (wheat), caffeine (coffee), licorice extract, cinnamon extract, green tea extract, lavender extract, lemon extract, and the like may be used. As the animal extracts, placenta (bovine placenta), royal jelly (honey bee secretion), snail extract (mucus secretion), and the like may be used.

A second aspect of the present application provides a method for preparing cerium oxide particles having surfaces modified with polyhydroxystearic acid, the method including: mixing polyhydroxystearic acid and a solvent to prepare a mixed solution; and mixing the mixed solution with cerium oxide particles and performing milling to prepare cerium oxide particles having surfaces modified with polyhydroxystearic acid.

Detailed description of the parts overlapping with the first aspect of the present application has been omitted, but the contents described for the first aspect of the present application may be equally applied even if the description thereof is omitted in the second aspect.

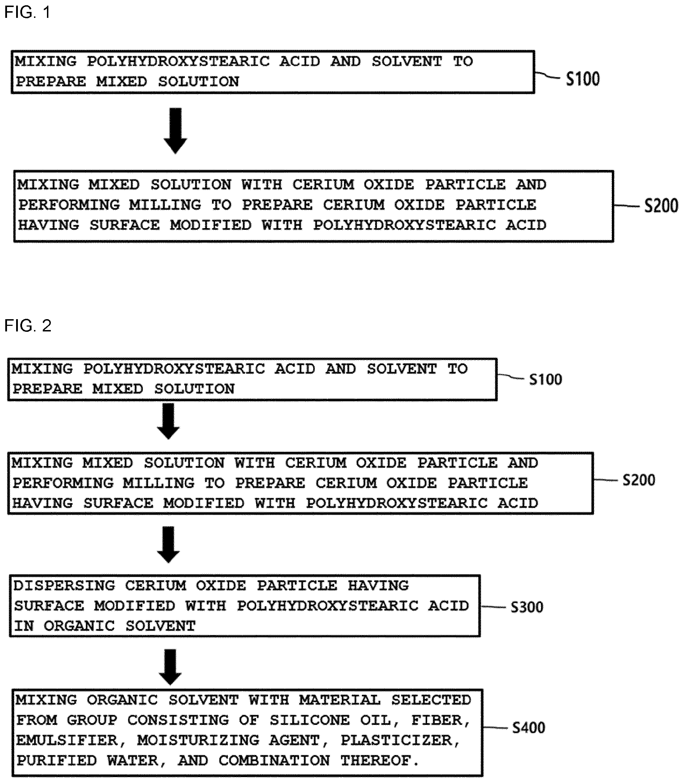

Hereinafter, the method for preparing cerium oxide particles having surfaces modified with polyhydroxystearic acid according to the second aspect of the present application will be described in detail with reference to FIG. 1.

First, in an embodiment of the present application, the method for preparing cerium oxide particles having surfaces modified with polyhydroxystearic acid includes a step (S100) of mixing polyhydroxystearic acid and a solvent to prepare a mixed solution.

In an embodiment of the present application, the solvent is not particularly limited as long as it is an organic solvent, and may be, for example, a solvent selected from the group consisting of alkyl benzoate, methylene chloride, xylene, dimethylformamide (DMF), nitrobenzene, methylethylketone, dimethicone, polydimethylsiloxane, cyclopentasiloxane, and combinations thereof.

In an embodiment of the present application, the mixing ratio of the polyhydroxystearic acid to the solvent may be 1:10 to 100. Polyhydroxystearic acid may be evenly dispersed in the solvent by mixing and stirring the polyhydroxystearic acid and the solvent as above. This makes it possible to evenly modify the surfaces of cerium oxide particles added thereafter with polyhydroxystearic acid.

Next, in an embodiment of the present application, the method for preparing cerium oxide particles having surfaces modified with polyhydroxystearic acid includes a step (S200) of mixing the mixed solution with cerium oxide particles and performing milling to prepare cerium oxide particles having surfaces modified with polyhydroxystearic acid.

In an embodiment of the present application, the particle size of cerium oxide may be 0.01 to 1 µm, preferably 0.05 to 0.5 µm, more preferably 0.08 to 0.2 µm. The mixing ratio of the mixed solution to the cerium oxide particles may be 1:0.5 to 1.5.

In an embodiment of the present application, mixing of the mixed solution with cerium oxide particles and milling may be performed at 20°C to 50°C, preferably at 25°C to 35°C, and the mixing and milling times may be 10 minutes to 180 minutes. At this time, the milling may be performed using a bead mill.

In an embodiment of the present application, before mixing of the mixed solution with cerium oxide particles and milling are performed, the method may further include removing impurities in the solution. A centrifugal separator and the like may be used to remove impurities, and there is no particular limitation on the method for removing impurities.

In an embodiment of the present application, after mixing of the mixed solution with cerium oxide particles and milling are performed, the cerium oxide particles having surfaces modified with polyhydroxystearic acid may be diluted to have a desired content.

A third aspect of the present application provides a method for preparing a sunscreen composition, the method including: dispersing the cerium oxide particles prepared in the second aspect to have surfaces modified with polyhydroxystearic acid in an organic solvent; and mixing the organic solvent with a material selected from the group consisting of silicone oil, a fiber, an emulsifier, a moisturizing agent, a plasticizer, purified water, and combinations thereof.

Detailed description of the parts overlapping with the first and second aspects of the present application has been omitted, but the contents described for the first and second aspects of the present application may be equally applied even if the description thereof is omitted in the third aspect.

Hereinafter, the method for preparing a sunscreen composition according to the third aspect of the present application will be described in detail with reference to FIG. 2.

First, in an embodiment of the present application, the method for preparing a sunscreen composition includes a step (S300) of dispersing the cerium oxide particles prepared in the second aspect to have surfaces modified with polyhydroxystearic acid in an organic solvent.

In an embodiment of the present application, the step of dispersing the cerium oxide particles having surfaces modified with polyhydroxystearic acid in an organic solvent is a process for evenly dispersing the cerium oxide particles exhibiting increased emulsifiability/dispersibility by surface modification in an organic solvent. At this time, it is preferable that alcohols are used as the organic solvent, and ethyl alcohol, behenyl alcohol, phenethyl alcohol, glycol, derivatives thereof, and the like may be used as the alcohols.

Next, in an embodiment of the present application, the method for preparing a sunscreen composition includes a step (S400) of mixing the organic solvent with a material selected from the group consisting of silicone oil, a fiber, an emulsifier, a moisturizing agent, a plasticizer, purified water, and combinations thereof.

In an embodiment of the present application, the step of mixing the organic solvent with a material selected from the group consisting of silicone oil, a fiber, an emulsifier, a moisturizing agent, a plasticizer, purified water, and combinations thereof is a step of preparing a formulation that can be used as a cosmetic composition by mixing cerium oxide particles dispersed in an organic solvent with various mixtures for cosmetic composition preparation. At this time, the kinds of organic solvents, silicone oils, fibers, emulsifiers, moisturizing agents, and plasticizers that can be used are as described in the first aspect of the present application.

### [Examples]

Hereinafter, Examples of the present invention will be described in detail so that those skilled in the technical field to which the present invention pertains can easily implement the present invention. However, the present invention may be implemented in various different forms and is not limited to Examples described herein.

### Preparation Example Preparation of cerium oxide particle

In order to prepare the sunscreen composition according to the present invention, cerium oxide particles were prepared.

Cerium hydroxide, cerium carbonate, cerium nitrate, cerium chloride, and ammonium cerium nitrate were mixed together and blended, and then subjected to a heat treatment at 400°C to 1200°C to obtain cerium oxide particles.

### Example Preparation of sunscreen composition containing cerium oxide particle having surface modified with polyhydroxystearic acid

### Step 1: Preparation of cerium oxide particle having surface modified with polyhydroxystearic acid

To 2,500 g of alkyl benzoate, 50 g of polyhydroxystearic acid was added, and then the mixture was stirred. To the prepared solution, 2,500 g of the cerium oxide particles was added, then milling was performed using a bead mill, and the mixture was diluted to obtain a dispersion of cerium oxide particles having surfaces modified with polyhydroxystearic acid.

### Step 2: Preparation of sunscreen composition

A cerium oxide solution was prepared by dispersing 200 g of the surface-modified cerium oxide dispersion obtained in step 1 at 20% by weight in 800 g of a mixed alcohol solvent containing glycol, behenyl alcohol, ethanol, and purified water at a ratio of 1:1:2:8. Thereafter, the prepared solution (54.55 wt%), silicone oil (DC 245, 10 wt%), a fiber (VGL silk, 10 wt%), a PEG silicone emulsifier (KF 6017, 3 wt%), a nonionic W/O emulsifier (Abil em 90, 2.5 wt%), a moisturizing agent (1,2-hexanediol, 2 wt%), a plasticizer (DPG, 10 wt%), and purified water (7.95 wt%) were mixed together to prepare a sunscreen composition.

### Comparative Example Preparation of sunscreen composition containing cerium oxide particle having surface modified with fatty acid

A sunscreen composition was prepared using cerium oxide particles having surfaces modified with stearic acid.

### Step 1: Preparation of cerium oxide particle having surface modified with stearic acid

First, 5 g of cerium oxide having a particle size of 110 nm prepared in Preparation Example was added to 150 g of purified water and then dispersed through ultrasonic agitation for 24 hours, and the supernatant was recovered.

Next, sodium stearate was dissolved in the supernatant at a molar ratio of 1:1.5, and 0.1 N NaOH as an acidity adjusting agent was added to the mixed solution to adjust the pH to 10.5.

Next, high-speed stirring was performed at 45°C for 24 hours. After the stirring was terminated, sodium and residual impurities in the solution were removed by washing with water three times through centrifugation.

Finally, a negative pressure was formed in a vacuum oven, and vacuum hot air drying was performed at 50°C for 24 hours to obtain a surface-modified cerium oxide solid.

### Step 2: Preparation of sunscreen composition

A cerium oxide solution was prepared by dispersing 200 g of the surface-modified cerium oxide solid obtained in step 1 at 20% by weight in 800 g of a mixed alcohol solvent containing glycol, behenyl alcohol, ethanol, and purified water at a ratio of 1:1:2:8. Thereafter, the prepared solution (54.55 wt%), silicone oil (DC 245, 10 wt%), a fiber (VGL silk, 10 wt%), a PEG silicone emulsifier (KF 6017, 3 wt%), a nonionic W/O emulsifier (Abil em 90, 2.5 wt%), a moisturizing agent (1,2-hexanediol, 2 wt%), a plasticizer (DPG, 10 wt%), and purified water (7.95 wt%) were mixed together to prepare a sunscreen composition.

### Experimental Example 1 Experiment to measure dispersion stability

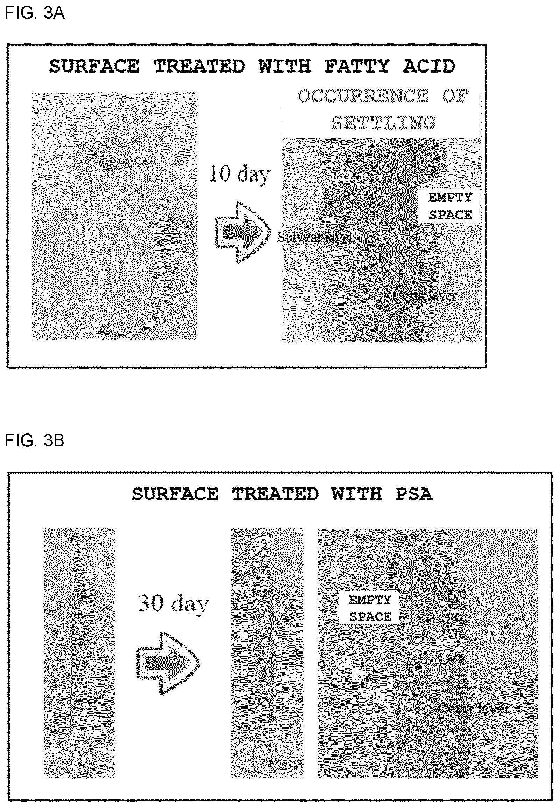

In order to test the dispersion stability of the sunscreen compositions of Comparative Example and Example, the prepared sunscreen compositions were left for a long period of time, and then it was observed whether layer separation between the solvent and the cerium oxide layer occurred. The photographs of the sunscreen compositions are illustrated in FIGS. 3A and 3B, respectively.

As illustrated in FIGS. 3A and 3B, it has been confirmed that layer separation did not occur even after 30 days has elapsed in the case of a sunscreen composition containing cerium oxide particles having surfaces modified with polyhydroxystearic acid while layer separation between the solvent and the cerium oxide layer occured after 10 days has elapsed in the case of a sunscreen composition containing cerium oxide particles having surfaces modified with a fatty acid. Hence, it has been confirmed that the sunscreen composition containing cerium oxide particles having surfaces modified with polyhydroxystearic acid corresponding to Example of the present invention can maintain excellent dispersion stability even when being left for a long period of time as compared to the sunscreen composition containing cerium oxide particles having surfaces modified with a fatty acid corresponding to Comparative Example.

### Experimental Example 2 Analysis of particle size distribution of cerium oxide particle having surface modified with polyhydroxystearic acid

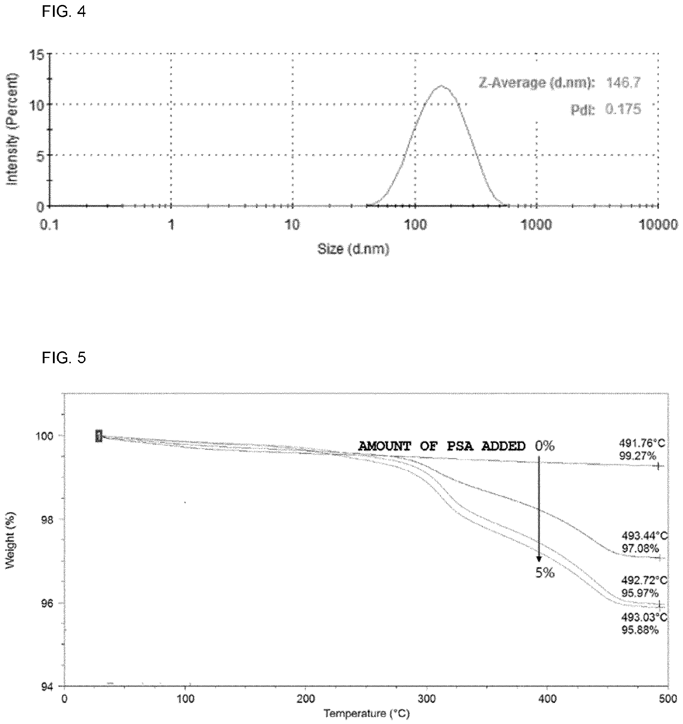

The particle size distribution of the cerium oxide particles having surfaces modified with polyhydroxystearic acid according to Example of the present invention was analyzed and illustrated in FIG. 4.

As illustrated in FIG. 4, it has been confirmed that the particle sizes of the cerium oxide particles having surfaces modified with polyhydroxystearic acid are most distributed in a range of 100 nm to 500 nm.

### Experimental Example 3 Thermogravimetric analysis (TGA) of cerium oxide particle depending on amount of polyhydroxystearic acid added

The thermogravimetric analysis of the cerium oxide particles having surfaces modified with polyhydroxystearic acid according to Example of the present invention was performed, and the results are illustrated in FIG. 5. As illustrated in FIG. 5, it has been confirmed that the decrease in weight increases as the added amount of polyhydroxystearic acid increases. It has been determined that this is because polyhydroxystearic acid that is a hydrophobic polymer is carbonized at high temperatures.

## Claims

1. A sunscreen composition comprising cerium oxide (CeO₂) particles having surfaces modified with substituted or unsubstituted polyhydroxystearic acid.

2. The sunscreen composition according to claim 1, wherein the polyhydroxystearic acid has a hydroxystearic acid repeating unit.

3. The sunscreen composition according to claim 1, wherein the polyhydroxystearic acid has a weight average molecular weight of 1,000 g/mol to 100,000 g/mol.

4. The sunscreen composition according to claim 1, wherein a content of the polyhydroxystearic acid is 1 part by weight to 10 parts by weight with respect to 100 parts by weight of cerium oxide particles.

5. The sunscreen composition according to claim 1, wherein a content of the surface-modified cerium oxide particles is 1 part by weight to 50 parts by weight with respect to 100 parts by weight of the sunscreen composition.

6. The sunscreen composition according to claim 1, wherein a primary particle size of the cerium oxide is 10 to 30 nm,
a secondary particle size of the cerium oxide is 100 to 500 nm, and
a ratio of the secondary particle size to the primary particle size is 5 to 50.

7. The sunscreen composition according to claim 1, which further comprises one or more kinds of particles selected from the group consisting of titanium oxide particles and zinc oxide particles.

8. The sunscreen composition according to claim 7, wherein a content of the titanium oxide particles or zinc oxide particles is 1 part by weight to 25 parts by weight with respect to 100 parts by weight of the sunscreen composition.

9. A method for preparing cerium oxide particles having surfaces modified with polyhydroxystearic acid, the method comprising:
mixing polyhydroxystearic acid and a solvent to prepare a mixed solution; and
mixing the mixed solution with cerium oxide particles and performing milling to prepare cerium oxide particles having surfaces modified with polyhydroxystearic acid.

10. The method for preparing cerium oxide particles having surfaces modified with polyhydroxystearic acid according to claim 9, wherein the solvent is a solvent selected from the group consisting of alkyl benzoate, methylene chloride, xylene, dimethylformamide (DMF), nitrobenzene, methylethylketone, dimethicone, polydimethylsiloxane, cyclopentasiloxane, and combinations thereof.

11. The method for preparing cerium oxide particles having surfaces modified with polyhydroxystearic acid according to claim 9, wherein a mixing ratio of the polyhydroxystearic acid to the solvent is 1:10 to 100.

12. The method for preparing cerium oxide particles having surfaces modified with polyhydroxystearic acid according to claim 9, wherein a mixing ratio of the mixed solution to the cerium oxide particles is 1:0.5 to 1.5.

13. A method for preparing a sunscreen composition, the method comprising:
dispersing the cerium oxide particles prepared in claim 9 to have surfaces modified with polyhydroxystearic acid in an organic solvent; and
mixing the organic solvent with a material selected from the group consisting of silicone oil, a fiber, an emulsifier, a moisturizing agent, a plasticizer, purified water, and combinations thereof.
